# FASCICULE DE BREVET EUROPEEN

(11) **EP 4 098 299 B1**
(45) Date de publication et mention de la délivrance du brevet: **16.07.2025**
(21) Numéro de dépôt: 22176527.4
(22) Date de dépôt: 31.05.2022
(51) Int. Cl.: A61M 5/31, A61M 5/32

(54) **DISPOSITIF DE PROTECTION D'AIGUILLE POUR SERINGUE**
SCHUTZVORRICHTUNG FÜR DIE SPRITZENNADEL
SYRINGE NEEDLE PROTECTION DEVICE

(30) Priorité: 31.05.2021 FR 2105696
(43) Date de publication de la demande: 07.12.2022
(73) Titulaire: Aptar Stelmi SAS, 93420 Villepinte (FR)
(72) Inventeur: SWAL, Mickaël, 60800 SERY MAGNEVAL (FR); JOUBERT, Françoise, 26150 DIE (FR)
(74) Mandataire: CAPRI

(56) Documents cités:
- WO-A2-02/074367
- FR-A1- 2 816 848
- FR-A1- 3 011 473
- US-A1- 2004 111 066
- US-A1- 2015 174 337

## Description

La présente invention concerne un dispositif de protection d'aiguille et un ensemble de distribution comportant une seringue et un tel dispositif de protection d'aiguille.

Des dispositifs de protection d'aiguille de seringue, également appelés protèges-aiguilles, sont connus, notamment des documents EP0429052, EP0976415, EP1208861, WO2015052417, WO2017051108, FR2816848, FR3011473, US2015174337, WO02074367 et US2004111066.

Toutefois, ce type de dispositif de protection peut présenter des inconvénients.

Ainsi, selon le matériau utilisé pour la partie souple du dispositif de protection, la force de retrait nécessaire pour retirer le dispositif de protection peut s'avérer trop faible, typiquement inférieure à 5N. Ceci peut présenter des risques de fiabilité.

La présente invention a pour objectif de fournir un dispositif de protection d'aiguille ne présentant pas les inconvénients précités.

Plus particulièrement, la présente invention a pour but de fournir un dispositif de protection d'aiguille qui requiert une force de retrait supérieure à 5N.

La présente invention a aussi pour but de fournir un tel dispositif de protection d'aiguille qui soit simple et peu coûteux à fabriquer et à assembler, et fiable d'utilisation.

La présente invention a donc pour objet un dispositif de protection d'aiguille comportant un capuchon élastique s'étendant axialement entre une extrémité arrière ouverte et une extrémité avant fermée, ledit capuchon élastique définissant une cavité interne délimitée par une paroi latérale et par une paroi radiale d'extrémité, ladite paroi d'extrémité recevant de manière étanche, en position assemblée, une pointe d'une aiguille d'une seringue, ladite cavité interne comportant un premier tronçon relié à une ouverture de ladite extrémité arrière dudit capuchon, un deuxième tronçon relié audit premier tronçon, ledit deuxième tronçon étant cylindrique et ayant un diamètre interne Dcav, et un troisième tronçon relié audit deuxième tronçon, une projection annulaire radiale étant disposée entre lesdits premier et deuxième tronçons, ladite projection annulaire ayant un diamètre interne Dproj et coopérant de manière étanche avec une partie avant du corps de ladite seringue lorsque ledit dispositif de protection est assemblé sur une seringue, de sorte que ladite projection annulaire forme une marche ayant une dimension radiale d correspondant à la différence entre Dcav et Dproj, dans lequel ledit diamètre interne Dcav dudit deuxième tronçon est compris entre 3.5 mm et 4.1 mm, ledit diamètre interne Dproj de ladite projection annulaire radiale est compris entre 3.0 mm et 3.6 mm, ladite dimension radiale d est supérieure ou égale à 0.50 mm, et ledit rapport d/Dcav est supérieur ou égal à 12%, pour générer une force de retrait dudit dispositif de protection d'au moins 5.5 N.

Avantageusement, ledit troisième tronçon est conique en se rétrécissant en direction de ladite paroi d'extrémité.

Avantageusement, un quatrième tronçon est relié audit troisième tronçon et se termine dans ladite paroi d'extrémité.

Avantageusement, ledit quatrième tronçon est cylindrique de section circulaire et présente un diamètre sensiblement égal au diamètre externe d'une aiguille de seringue.

Avantageusement, le dispositif comporte une coque rigide dans laquelle est logé ledit capuchon élastique.

Avantageusement, ladite projection radiale annulaire comporte au moins une fente.

La présente invention a aussi pour objet un ensemble de distribution comportant une seringue comprenant un corps pourvu d'une partie avant supportant une aiguille pourvue d'une pointe, et un dispositif de protection d'aiguille amovible tel que décrit ci-dessus, fixé de manière amovible sur ladite seringue.

Avantageusement, à l'état assemblé sur ladite seringue, ledit deuxième tronçon reçoit ladite partie avant de la seringue.

Ces caractéristiques et avantages et d'autres de la présente invention apparaîtront plus clairement au cours de la description détaillée suivante, faite en référence aux dessins joints, donnés à titre d'exemples non-limitatifs, et sur lesquels :
la figure 1 est une vue schématique en perspective découpée d'un ensemble de distribution comportant une seringue et un dispositif de protection d'aiguille, et
la figure 2 est une vue schématique en section transversale d'un dispositif de protection d'aiguille selon un mode de réalisation avantageux de la présente invention.

Dans la description qui suit, les termes "axial" et "radial" se réfèrent à l'axe central longitudinal A du dispositif.

Sur les figures, il est représenté un dispositif de protection 10 pour aiguille de seringue, ou protège-aiguille. Ce dispositif de protection 10 est destiné à être assemblé de manière amovible sur une seringue 20.

Une telle seringue 20 comporte un corps de seringue 21 contenant un piston (non représenté), la partie avant 22 dudit corps de seringue 21 supportant une aiguille 23 pourvue d'une pointe 24 définissant l'ouverture de distribution de l'aiguille A. Cette partie avant 22 est aussi appelée boule de la seringue, et c'est ce terme qui sera utilisé ci-après.

Le protège-aiguille 10 comporte un capuchon élastique 11 s'étendant selon une direction axiale longitudinale entre une extrémité arrière ouverte et une extrémité avant fermée. Le capuchon élastique 11 définit une cavité interne 12 délimitée par une paroi latérale 13 et par une paroi radiale d'extrémité 14. Le capuchon élastique 11 est réalisé en matériau souple, typiquement du caoutchouc.

La cavité interne 12 est composé de plusieurs tronçons s'étendant depuis une ouverture 15 située à l'extrémité arrière ouverte du capuchon 11, jusqu'à la paroi d'extrémité 14.

De manière adjacente à l'ouverture 15, la cavité interne 12 comporte un premier tronçon 12a qui présente une forme cylindrique de section circulaire sur les figures. **Il** est à noter qu'une forme tronconique allant en se rétrécissant en direction de la paroi d'extrémité 14 pourrait toutefois également être utilisée.

Tel qu'illustré, le premier tronçon 12a présente un diamètre sensiblement égal au diamètre de l'ouverture 15.

Ce premier tronçon 12a se prolonge par un deuxième tronçon 12b destiné à recevoir la boule 22 de la seringue 20 lorsque le protège-aiguille 10 est assemblé sur la seringue 20, ce deuxième tronçon 12b présentant une forme cylindrique de section circulaire et présentant un diamètre interne Dcav inférieur au diamètre de l'ouverture 15.

Ce deuxième tronçon 12b se prolonge à son tour par un troisième tronçon 12c dont le diamètre va en se rétrécissant progressivement en direction de la paroi d'extrémité 14.

A proximité de cette paroi d'extrémité 14, ce troisième tronçon 12c se prolonge par un quatrième tronçon 12d cylindrique de section circulaire présentant un diamètre sensiblement égal au diamètre externe de l'aiguille 23 de la seringue 20.

Entre les premier et deuxième tronçons 12a et 12b de la cavité interne 12, il est prévu une projection annulaire radiale 16, tel qu'un bourrelet, formant un renflement interne de matière. Cette projection annulaire 16 présente un diamètre interne Dproj inférieur au diamètre Dcav du second tronçon 12b de la cavité interne 12.

Cette projection 16 forme une marche ayant une dimension radiale d correspondant à la différence entre Dcav et Dproj. Elle forme également un cordon de retenue mécanique. Lors du passage en autoclave, elle améliore la retenue de la boule 22 de la seringue 20 dans le logement 12 du capuchon élastique 11.

Afin de faciliter, lors du passage en autoclave, le passage de la vapeur d'eau sous pression hors de la cavité interne 12 et d'améliorer la déformabilité de cette projection annulaire 16, celle-ci est munie d'une pluralité de fentes 17, avantageusement quatre, s'étendant en direction axiale, de préférence de manière rectiligne.

On peut bien entendu prévoir un nombre quelconque de fentes 17, lesquelles peuvent présenter une profondeur plus ou moins importante. Si elles sont en grand nombre, ces fentes 17 séparent entre eux un grand nombre de portions de la projection 16 qui forment chacun une petite protubérance. De même, ces fentes 17 peuvent être plus ou moins larges.

Dans l'exemple de réalisation représenté sur les figures, le dispositif de protection 10 pour aiguille de seringue comporte, outre le capuchon élastique 11, une coque rigide 19 dans laquelle est logé le capuchon élastique 11. La présence de cette coque rigide 19 n'est toutefois pas obligatoire pour la présente invention.

Ce type de coque 19 est classiquement utilisé pour renforcer la protection de l'utilisateur de la seringue contre une piqûre par l'aiguille en offrant une protection supplémentaire extérieure rigide qui est difficilement percée par l'aiguille 23. Cette coque rigide 19 est montée de manière coaxiale par rapport au capuchon 11. La coque rigide 19 est dimensionnée pour permettre l'insertion et le blocage du capuchon 11. A cet effet, la coque rigide 19 présente une forme intérieure qui suit sensiblement la forme extérieure du capuchon 11. Afin de retenir le capuchon 11 à l'intérieur de la coque rigide 19, on prévoit avantageusement des moyens de retenue du capuchon 11, qui peuvent comprendre un rebord rentrant 18, de préférence annulaire.

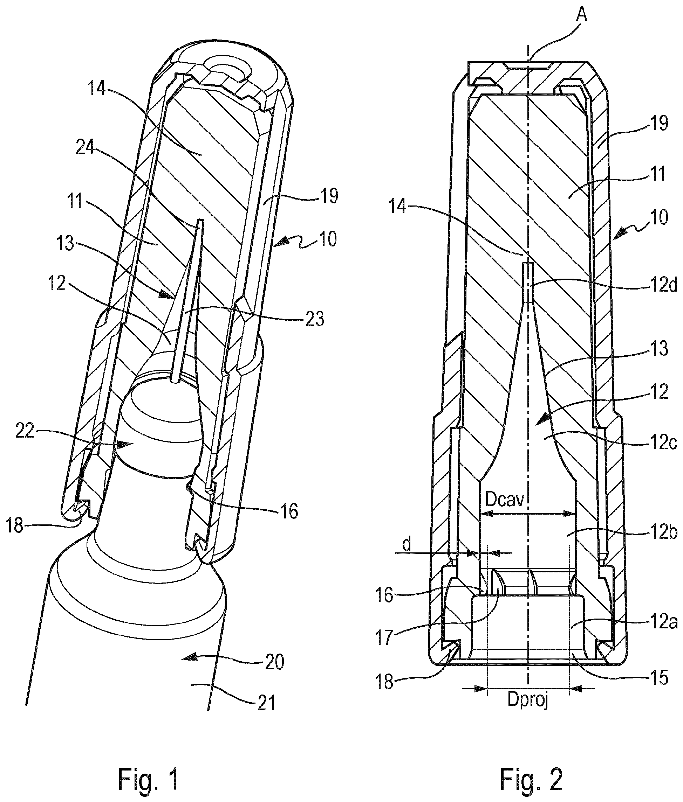

Comme illustré sur la figure 1, lorsque le protège-aiguille 10 est en position de protection autour de l'aiguille 23, l'extrémité libre, c'est-à-dire la pointe 24, de l'aiguille 23 est plantée dans la paroi d'extrémité 14 du capuchon 11 tandis que la boule 22 de la seringue 20 pénètre dans la cavité interne 12 du capuchon 11, et coopère de manière étanche avec ladite projection annulaire 16.

De manière connue notamment du document EP1208861, la projection annulaire 16 coopère avec une paroi latérale de la boule 22 de la seringue 20, ce qui réalise la fermeture étanche de ladite cavité interne 12 par rapport à l'atmosphère. Au moment de l'insertion de la boule 22 du corps 21 de la seringue 20 dans la cavité interne 12 du capuchon 11, il y a un écrasement de la projection annulaire 16 par la boule 22 de la seringue, et les fentes 17 n'empêchent donc pas la fermeture étanche de la cavité 12.

Un but de la présente invention est de garantir une force de retrait du dispositif de protection d'aiguille d'au moins 5,5N.

Des tests comparatifs de plusieurs géométries internes ont révélés de manière surprenante que le paramètre principal pour augmenter cette force de retrait n'est pas le serrage du capuchon élastique 11 sur la boule 22 de la seringue, mais l'importance de la "marche" formée entre la partie du capuchon recevant ladite boule, à savoir le deuxième tronçon 12b, et la projection annulaire 16.

Le tableau ci-après illustre ces tests comparatifs :

| **Résultats forces de retrait Protège Aiguille en fonction des géométries** | | | | |
|---|---|---|---|---|
| ***Dcav (mm)*** | ***Dproj (mm)*** | ***d (mm)*** | ***Force de retrait (N)*** | ***d*/*Dcav (%)*** |
| 3.80 | 3.60 | 0.20 | 4.50 | 5.3 |
| 4.00 | 3.60 | 0.40 | 5.00 | 10.0 |
| 3.60 | 3.20 | 0.40 | 5.15 | 11.1 |
| 4.10 | 3.60 | 0.50 | 5.50 | 12.2 |
| 3.50 | 3.00 | 0.50 | 5.95 | 14.3 |
| 4.00 | 3.40 | 0.60 | 6.70 | 15.0 |

De ces résultats, on observe que plus le rapport d/Dcav est important, plus les forces de retrait augmentent. C'est ce rapport qui forme le paramètre le plus important pour définir la force de retrait.

Ainsi, une simple augmentation du serrage du capuchon sur la boule 22 de la seringue, par une diminution des diamètres Dcav et Dproj, ne suffit pas pour augmenter la force de retrait. En effet, la configuration Dcav = 3.5 mm et Dproj = 3.0 mm génère une force de retrait inférieure à la configuration Dcav = 4.0 mm et Dproj = 3.4 mm. De même, la configuration Dcav = 3.6 mm et Dproj = 3.2 mm génère une force de retrait inférieure à la configuration Dcav = 4.1 mm et Dproj = 3.6 mm.

De plus, à dimension d identique, c'est toujours la configuration avec le rapport d/Dcav supérieur qui génère une force de retrait supérieure.

Selon l'invention, le diamètre interne Dcav du deuxième tronçon 12b de la cavité interne 12 est compris entre 3.5 et 4.1 mm, le diamètre interne Dproj de la projection annulaire 16 est compris entre 3.0 et 3.6 mm, le rapport d/Dcav est supérieur ou égal à 12% et la dimension d est supérieure ou égale à 0.50 mm, ce qui assure une force de retrait d'au moins 5,5N, comme démontré par les tests comparatifs.

Bien que la présente invention ait été décrite en référence à un mode de réalisation particulier, il est entendu que la présente invention n'est pas limitée par celui-ci mais qu'au contraire l'homme du métier peut y apporter toutes modifications utiles sans sortir du cadre de la présente invention tel que défini par les revendications annexées.

## Revendications

1. Dispositif de protection d'aiguille (10) comportant un capuchon élastique (11) s'étendant axialement entre une extrémité arrière ouverte et une extrémité avant fermée, ledit capuchon élastique (11) définissant une cavité interne (12) délimitée par une paroi latérale (13) et par une paroi radiale d'extrémité (14), ladite paroi d'extrémité (14) recevant de manière étanche, en position assemblée, une pointe (24) d'une aiguille (23) d'une seringue (20), ladite cavité interne (12) comportant un premier tronçon (12a) relié à une ouverture (15) de ladite extrémité arrière dudit capuchon (11), un deuxième tronçon (12b) relié audit premier tronçon (12a), ledit deuxième tronçon étant cylindrique et ayant un diamètre interne (Dcav), et un troisième tronçon (12c) relié audit deuxième tronçon (12b), une projection annulaire radiale (16) étant disposée entre lesdits premier et deuxième tronçons (12a, 12b), ladite projection annulaire (16) ayant un diamètre interne (Dproj) et coopérant de manière étanche avec une partie avant (22) du corps (21) de ladite seringue (20) lorsque ledit dispositif de protection (10) est assemblé sur une seringue (20), de sorte que ladite projection annulaire (16) forme une marche ayant une dimension radiale (d) correspondant à la différence entre Dcav et Dproj, **caractérisé en ce que** ledit diamètre interne (Dcav) dudit deuxième tronçon (12b) est compris entre 3.5 mm et 4.1 mm, ledit diamètre interne (Dproj) de ladite projection annulaire radiale (16) est compris entre 3.0 mm et 3.6 mm, ladite dimension radiale (d) est supérieure ou égale à 0.50 mm, et ledit rapport (d/Dcav) est supérieur ou égal à 12%, pour générer une force de retrait dudit dispositif de protection (10) d'au moins 5.5 N.

2. Dispositif selon la revendication 1, dans lequel ledit troisième tronçon (12c) est conique en se rétrécissant en direction de ladite paroi d'extrémité (14).

3. Dispositif selon la revendication 1 ou 2, dans lequel un quatrième tronçon (12d) est relié audit troisième tronçon (12c) et se termine dans ladite paroi d'extrémité (14).

4. Dispositif selon la revendication 3, dans lequel ledit quatrième tronçon (12d) est cylindrique de section circulaire et présente un diamètre sensiblement égal au diamètre externe d'une aiguille (23) de seringue.

5. Dispositif selon l'une quelconque des revendications précédentes, comportant une coque rigide (19) dans laquelle est logé ledit capuchon élastique (11).

6. Dispositif selon l'une quelconque des revendications précédentes, dans lequel ladite projection radiale annulaire (16) comporte au moins une fente (17).

7. Ensemble de distribution comportant une seringue (20) comprenant un corps (21) pourvu d'une partie avant (22) supportant une aiguille (23) pourvue d'une pointe (24), et un dispositif de protection d'aiguille (10) selon l'une quelconque des revendications précédentes, fixé de manière amovible sur ladite seringue (20).

8. Ensemble selon la revendication 7, dans lequel, à l'état assemblé sur ladite seringue (20), ledit deuxième tronçon (12b) est configuré pour recevoir ladite partie avant (22) de la seringue.

## Patentansprüche

1. Nadelschutzvorrichtung (10) mit einer elastischen Kappe (11), die sich axial zwischen einem offenen hinteren Ende und einem geschlossenen vorderen Ende erstreckt, wobei die elastische Kappe (11) einen inneren Hohlraum (12) definiert, der von einer Seitenwand (13) und einer radialen Endwand (14) begrenzt wird, wobei die Endwand (14) in zusammengebauter Position eine Spitze (24) einer Nadel (23) einer Spritze (20) dicht aufnimmt, wobei der innere Hohlraum (12) einen ersten Abschnitt (12a) aufweist, der mit einer Öffnung (15) des hinteren Endes der Kappe (11) verbunden ist, einen zweiten Abschnitt (12b), der mit dem ersten Abschnitt (12a) verbunden ist, wobei der zweite Abschnitt zylindrisch ist und einen Innendurchmesser (Dcav) aufweist, und einen dritten Abschnitt (12c), der mit dem zweiten Abschnitt (12b) verbunden ist, wobei ein radialer ringförmiger Vorsprung (16) zwischen dem ersten und zweiten Abschnitt (12a, 12b) angeordnet ist, wobei der ringförmige Vorsprung (16) einen Innendurchmesser (Dproj) aufweist und dicht mit einem vorderen Teil (22) des Körpers (21) der Spritze (20) zusammenwirkt, wenn die Schutzvorrichtung (10) auf einer Spritze (20) zusammengebaut ist, so dass der ringförmige Vorsprung (16) eine Stufe mit einer radialen Abmessung (d) bildet, die der Differenz zwischen Dcav und Dproj entspricht, **dadurch gekennzeichnet, dass** der Innendurchmesser (Dcav) des zweiten Abschnitts (12b) zwischen 3,5 mm und 4,1 mm liegt, der Innendurchmesser (Dproj) des radialen ringförmigen Vorsprungs (16) zwischen 3,0 mm und 3,6 mm liegt, wobei die radiale Abmessung (d) größer oder gleich 0,50 mm ist und das Verhältnis (d/Dcav) größer oder gleich 12% ist, um eine Rückzugskraft der Schutzvorrichtung (10) von mindestens 5,5 N zu erzeugen.

2. Vorrichtung nach Anspruch 1, wobei der dritte Abschnitt (12c) konisch ist, indem er sich in Richtung der Endwand (14) verjüngt.

3. Vorrichtung nach Anspruch 1 oder 2, wobei ein vierter Abschnitt (12d) mit dem dritten Abschnitt (12c) verbunden ist und in der Endwand (14) endet.

4. Vorrichtung nach Anspruch 3, wobei der vierte Abschnitt (12d) im Querschnitt zylindrisch ist und einen Durchmesser aufweist, der im Wesentlichen dem Außendurchmesser einer Spritzennadel (23) entspricht.

5. Vorrichtung nach einem der vorstehenden Ansprüche, mit einer starren Hülle (19), in der die elastische Kappe (11) untergebracht ist.

6. Vorrichtung nach einem der vorstehenden Ansprüche, wobei der ringförmige radiale Vorsprung (16) mindestens einen Schlitz (17) aufweist.

7. Abgabeanordnung, die eine Spritze (20) aufweist, die einen Körper (21) mit einem vorderen Teil (22) umfasst, der eine Nadel (23) mit einer Spitze (24) trägt, und eine Nadelschutzvorrichtung (10) nach einem der vorstehenden Ansprüche, die abnehmbar an der Spritze (20) befestigt ist.

8. Anordnung nach Anspruch 7, wobei im auf der Spritze (20) zusammengebauten Zustand der zweite Abschnitt (12b) ausgelegt ist, um den vorderen Teil (22) der Spritze aufzunehmen.

## Claims

1. A needle protection device (10) including an elastic cap (11) extending axially between an open rear end and a closed front end, said elastic cap (11) defining an inner cavity (12) delimited by a side wall (13) and by a radial end wall (14), said end wall (14) receiving in a sealing manner, in the assembled position, a tip (24) of a needle (23) of a syringe (20), said inner cavity (12) including a first segment (12a) connected to an opening (15) of said rear end of said cap (11), a second segment (12b) connected to said first segment (12a), said second segment being cylindrical and having an internal diameter (Dcav), and a third segment (12c) connected to said second segment (12b), a radial annular projection (16) being disposed between said first and second segments (12a, 12b), said annular projection (16) having an internal diameter (Dproj) and cooperating in a sealing manner with a front part (22) of the body (21) of said syringe (20) when said protection device (10) is assembled on a syringe (20), so that said annular projection (16) forms a step having a radial dimension (d) corresponding to the difference between Dcav and Dproj, **characterized in that** said internal diameter (Dcav) of said second segment (12b) is comprised between 3.5 mm and **4.1 mm,** said internal diameter (Dproj) of said radial annular projection (16) is comprised between 3.0 mm and 3.6 **mm,** said radial dimension (d) is greater than or equal to 0.50 **mm,** and said ratio (d/Dcav) is greater than or equal to 12%, to generate a force for withdrawing said protection device (10) of at least 5.5 N.

2. The device according to claim 1, wherein said third segment (12c) is conical by narrowing towards said end wall (14).

3. The device according to claim 1 or 2, wherein a fourth segment (12d) is connected to said third segment (12c) and terminates in said end wall (14).

4. The device according to claim 3, wherein said fourth segment (12d) is cylindrical with a circular section and has a diameter substantially equal to the external diameter of a syringe needle (23).

5. The device according to any one of the preceding claims, including a rigid shell (19) wherein said elastic cap (11) is housed.

6. The device according to any one of the preceding claims, wherein said annular radial projection (16) includes at least one slot (17).

7. A dispensing assembly including a syringe (20) comprising a body (21) provided with a front part (22) supporting a needle (23) provided with a tip (24), and a needle protection device (10) according to any one of the preceding claims, removably fixed to said syringe (20).

8. The assembly according to claim 7, wherein, in the assembled state on said syringe (20), said second segment (12b) is configured to receive said front part (22) of the syringe.
